# EUROPEAN PATENT APPLICATION

(11) **EP 4 620 501 A1**
(43) Date of publication of application: **24.09.2025**
(21) Application number: 23890646.5
(22) Date of filing: 06.11.2023
(51) Int. Cl.: A61M 11/00, A61M 15/00

(54) **MULTI-MODE ATOMIZATION DEVICE AND MODE-SWITCHING MEMBER THEREOF**

(30) Priority: 14.11.2022 US 202263425068 P
(71) Applicant: Microbase Technology Corp., Taoyuan City 334 (TW)
(72) Inventor: SANG, Chen-hsiang, Taoyuan City, Taiwan 334 (TW); HUNG, Chi-shan, Taoyuan City, Taiwan 334 (TW); LO, Kai-yao, Taoyuan City, Taiwan 334 (TW); CHOU, Nien-yao, Taoyuan City, Taiwan 334 (TW)
(74) Representative: Viering, Jentschura & Partner mbB Patent- und Rechtsanwälte
(86) International application number: PCT/CN2023/129836
(87) International publication number: WO 2024/104210

(57) **Abstract**

Provided are a multi-atomization device and a mode-switching member thereof. The mode-switching member comprises a transmission cable as well as a handheld holder and a connector which are connected to two opposite ends of the transmission cable, respectively. The handheld holder comprises a holder body and a plurality of first metal pads fixed to the holder body, and an imitation engaging slot exposing the plurality of first metal pads is formed in the holder body. The connector comprises an embedded body and a plurality of second metal pads fixed to the embedded body, and the embedded body is provided with a copying bonding surface exposing the plurality of second metal pads. The plurality of second metal pads are electrically coupled to the plurality of first metal pads by means of the transmission cable, respectively, such that the handheld holder and the connector are used for being connected to an atomizer and a fixed-point drive station, respectively, to jointly form an extension operation mode. Therefore, the handheld holder has the function of bearing the atomizer for handheld operation, such that a user can operate the atomizer more stably.

## Description

### FIELD OF THE INVENTION

The present invention relates to an atomization device, and more particularly to a multi-mode atomization device and a mode-switching member thereof.

### BACKGROUND OF THE INVENTION

A conventional atomization device can be divided into a handheld type and a fixed-point type according to the usage mode, but the conventional atomization device does not have a related mechanism that is configured for alternating between the above two types. Accordingly, the conventional atomization device is not conducive to being operated by users in different situations. Therefore, believing that the above-mentioned defects can be improved, the inventor dedicated much research, applied scientific principles, and finally proposed the present invention that is reasonably designed and that effectively improves on the above-mentioned defects.

### SUMMARY OF THE INVENTION

The purpose of embodiments in the present invention is to provide a multi-mode atomization device and a mode-switching member thereof for effectively improving on the issues associated with conventional atomization devices.

An embodiment of the present invention provides a multi-mode atomization device, characterized in that the multi-mode atomization device comprises: an atomizer comprising: a carrier having a liquid storage chamber, a matching surface located on one side of the liquid storage chamber, and an atomization outlet that is located on another side of the liquid storage chamber; a plurality of first contacts fixed to the carrier and exposed from the matching surface; an atomization module mounted on the carrier, wherein two opposite sides of the atomization module are in spatial communication with the liquid storage chamber and the atomization outlet, respectively; and a fixed-point drive station including an engaging slot and a plurality of second contacts exposed from the engaging slot; and a mode-switching member including: a transmission cable including a plurality of conductive wires and an insulating layer that covers the plurality of conductive wires; a handheld holder including: a seat integrally connected to one end of the insulating layer and having an imitation engaging slot; and a plurality of first metal pads fixed to the seat and exposed from the imitation engaging slot, wherein the plurality of first metal pads are electrically coupled to the plurality of conductive wires, respectively; and a connector including: an engaging body integrally connected to another end of the insulating layer and having an imitation matching surface; and a plurality of second metal pads fixed to the engaging body and exposed from the imitation matching surface, wherein the plurality of second metal pads are electrically coupled to the plurality of conductive wires, respectively, so that each of the first metal pads is electrically coupled to one of the second metal pads through one of the conductive wires; wherein the multi-mode atomization device is selectively in an upright operation mode by inserting the atomizer into the fixed-point drive station, or in an extension operation mode by respectively assembling the handheld holder and the connector of the mode-switching member to the atomizer and the fixed-point drive station; wherein, when the multi-mode atomization device is in the upright operation mode, the carrier is inserted into the engaging slot, and the first contacts respectively abut against the second contacts, so that the atomizer and the fixed-point drive station are capable of standing on a working surface through a center of gravity thereof; wherein, when the multi-mode atomization device is in the extension operation mode, the carrier is inserted into the imitation engaging slot, and the plurality of first contacts respectively abut against the plurality of first metal pads, and the engaging body is inserted into the engaging slot, and the plurality of second metal pads respectively abut against the plurality of second contacts, so that the atomizer is configured to be movable relative to the fixed-point drive station through the mode-switching member.

Preferably, the carrier has a positioning sheet adjacent to the matching surface, and the fixed-point drive station has a locking slot that is recessed in an inner bottom wall of the engaging slot and that is arranged adjacent to the plurality of second contacts, wherein, when the multi-mode atomization device is in the upright operation mode, the positioning sheet of the carrier is inserted into the locking slot of the fixed-point drive station.

Preferably, the fixed-point drive station includes a locking member assembled in the locking slot, wherein, when the multi-mode atomization device is in the upright operation mode, the positioning sheet is inserted into the locking slot and is engaged with the locking member for jointly being in a locked state, and the locking member is pressable to separate from the positioning sheet, thereby enabling the locking member and the positioning sheet to be changed from the locked state to an unlocked state.

Preferably, the handheld holder includes a locking ring that is connected to the seat and that is arranged adjacent to the plurality of first metal pads, and the connector includes an imitation positioning sheet connected to the engaging body, wherein, when the multi-mode atomization device is in the extension operation mode, the positioning sheet of the carrier is inserted into the locking ring of the handheld holder, and the imitation positioning sheet of the connector is inserted into the locking slot of the fixed-point drive station.

Preferably, the handheld holder includes an insertion slot that is connected to the seat and that is arranged adjacent to the plurality of first metal pads and a button that is assembled in the insertion slot, and the connector includes an imitation positioning sheet connected to the engaging body, wherein, when the multi-mode atomization device is in the extension operation mode, the imitation positioning sheet of the connector is inserted into the locking slot of the fixed-point drive station, the positioning sheet of the carrier is inserted into the insertion slot and is interlocked with the button, and the button is pressable to separate from the positioning sheet.

Preferably, the atomizer includes a plurality of buffer pads assembled on the matching surface, and each of the plurality of first contacts is a metal wire inserted into one of the plurality of buffer pads, wherein a part of each of the plurality of first contacts protrudes outward from a corresponding one of the plurality of buffer pads so as to be able to elastically abut against one of the plurality of second contacts or one of the plurality of first metal pads.

Preferably, the atomizer has a plurality of ribs formed on the matching surface, and any one of the plurality of buffer pads and the corresponding first contact is located between two of the plurality of ribs adjacent to each other, the fixed-point drive station has a plurality of guiding grooves recessed in an inner side wall of the engaging slot, and any one of the plurality of second contacts is located between two of the plurality of guiding grooves adjacent to each other, wherein, when the multi-mode atomization device is in the upright operation mode, the plurality of ribs are respectively inserted into the plurality of guiding grooves.

Preferably, the seat of the handheld holder has a plurality of imitation guiding grooves recessed in the imitation engaging slot, and any one of the plurality of first metal pads is located between two of the plurality of imitation guiding grooves adjacent to each other, wherein the engaging body of the connector includes two imitation ribs formed on the matching surface, and the plurality of second metal pads are located between the two imitation ribs, and wherein, when the multi-mode atomization device is in the extension operation mode, the plurality of ribs are respectively inserted into the plurality of imitation guiding grooves, and the two imitation ribs are respectively inserted into two of the plurality of guiding grooves.

Preferably, the carrier has two positioning blocks connected to the matching surface and arranged on the liquid storage chamber, and the fixed-point drive station has two positioning notches that are respectively recessed in two corners of the engaging slot and that are arranged away from the plurality of second contacts, wherein, when the multi-mode atomization device is in the upright operation mode, the two positioning blocks are respectively engaged with the two positioning notches.

Preferably, the seat of the handheld holder has two imitation positioning notches that are respectively recessed in two corners of the imitation engaging slot and that are arranged away from the plurality of first metal pads, wherein the engaging body has two imitation positioning blocks connected to the imitation matching surface, and wherein, when the multi-mode atomization device is in the extension operation mode, the two positioning blocks are respectively engaged in the two imitation positioning notches, and the two imitation positioning blocks are respectively engaged in the two positioning notches.

Preferably, the liquid storage chamber of the atomizer has a container and a sealing cap that is pivotally connected to the container and that rotatably closes the container, wherein, when the multi-mode atomization device is in the upright operation mode or the extension operation mode, the sealing cap is rotatable by at least 90 degrees relative to the container.

Preferably, the sealing cap is pivotally connected to the container along a rotation axis, wherein, when the multi-mode atomization device is in the upright operation mode, the rotation axis is located within the engaging slot of the fixed-point drive station.

Preferably, when the multi-mode atomization device is in the extension operation mode, the rotation axis is exposed from the imitation engaging slot of the handheld holder.

Preferably, the engaging body of the connector corresponds in geometric shape to and is detachably inserted into the imitation engaging slot of the handheld holder.

An embodiment of the present invention provides a mode-switching member of a multi-mode atomization device, characterized in that the mode-switching member of the multi-mode atomization device comprises: a transmission cable including a plurality of conductive wires and an insulating layer that covers the plurality of conductive wires; a handheld holder including: a seat integrally connected to one end of the insulating layer and having an imitation engaging slot; and a plurality of first metal pads fixed to the seat and exposed from the imitation engaging slot, wherein the plurality of first metal pads are electrically coupled to the plurality of conductive wires, respectively; and a connector including: an engaging body integrally connected to another end of the insulating layer and having an imitation matching surface; and a plurality of second metal pads fixed to the engaging body and exposed from the imitation matching surface, wherein the plurality of second metal pads are electrically coupled to the plurality of conductive wires, respectively, so that each of the plurality of first metal pads is electrically coupled to one of the plurality of second metal pads through one of the plurality of conductive wires; wherein the handheld holder and the connector of the mode-switching member are configured to allow an atomizer and a fixed-point drive station to be respectively connected thereto, so that the atomizer and the fixed-point drive station assembled to each other and in an upright operation mode are adjustable to be separated from each other for being in an extension operation mode.

Preferably, the engaging body of the connector corresponds in geometric shape to and is detachably inserted into the imitation engaging slot of the handheld holder.

Preferably, the handheld holder includes an insertion slot that is connected to the seat and that is arranged adjacent to the plurality of first metal pads and a button that is assembled in the insertion slot, and the connector includes an imitation positioning sheet connected to the engaging body.

In summary, the multi-mode atomization device disclosed in the embodiments of the present invention can selectively use the mode-switching member to facilitate switching between the upright operation mode and the extension operation mode according to different requirements of the user. Furthermore, the mode-switching member of the multi-mode atomization device can also imitate part of the appearance of the fixed-point drive station through the handheld holder, thereby providing the carrying function and the hand-holding function for the atomizer for enabling the user to operate the atomizer more stably.

In order to further understand the features and technical content of the present invention, please refer to the following detailed description and drawings of the present invention. However, these descriptions and drawings are only used to illustrate the present invention and do not limit the scope of the present invention in any way.

### BRIEF DESCRIPTION OF THE DRAWINGS

FIG. 1 is a schematic perspective view of a multi-mode atomization device in an extension operation mode device according to a first embodiment of the present invention.
FIG. 2 is a schematic perspective view of the multi-mode atomization device in an upright operation mode device according to the first embodiment of the present invention.
FIG. 3 is a schematic exploded view of FIG. 2.
FIG. 4 is a schematic enlarged view of part IV of FIG. 3.
FIG. 5 is a schematic exploded view of FIG. 1.
FIG. 6 is a schematic enlarged view of part VI of FIG. 5.
FIG. 7 is a schematic enlarged view of part VII of FIG. 5.
FIG. 8 is a schematic perspective view showing a mode-switching member of FIG. 5 that has a handheld holder and a connector connected to the handheld holder.
FIG. 9 is a schematic perspective view of the multi-mode atomization device in the extension operation mode device according to a second embodiment of the present invention.
FIG. 10 is a schematic exploded view of FIG. 9.

### DETAILED DESCRIPTION OF THE EXEMPLARY EMBODIMENTS

The following is a specific example to illustrate the implementation of the "a multi-mode atomization device and a mode-switching member thereof" disclosed in the present invention. Those skilled in the art can understand the advantages and effects of the present invention from the content disclosed in this specification. The present invention can be implemented or applied through other different specific embodiments, and various details in this specification can also be modified and changed based on different viewpoints and applications without departing from the concept of the present invention. In addition, the drawings of the present invention are only simple schematic illustrations and are not depictions based on actual dimensions, as is stated in advance. The following embodiments will further describe the relevant technical content of the present invention in detail, but the disclosed content is not intended to limit the scope of the present invention.

It should be understood that although terms such as "first", "second" and "third" may be used herein to describe various components or signals, these components or signals should not be limited by these terms. These terms are primarily used to distinguish one component from another component or one signal from another signal. In addition, the term "or" used in this article shall include any one or combination of more of the associated listed items depending on the actual situation.

### [First embodiment]

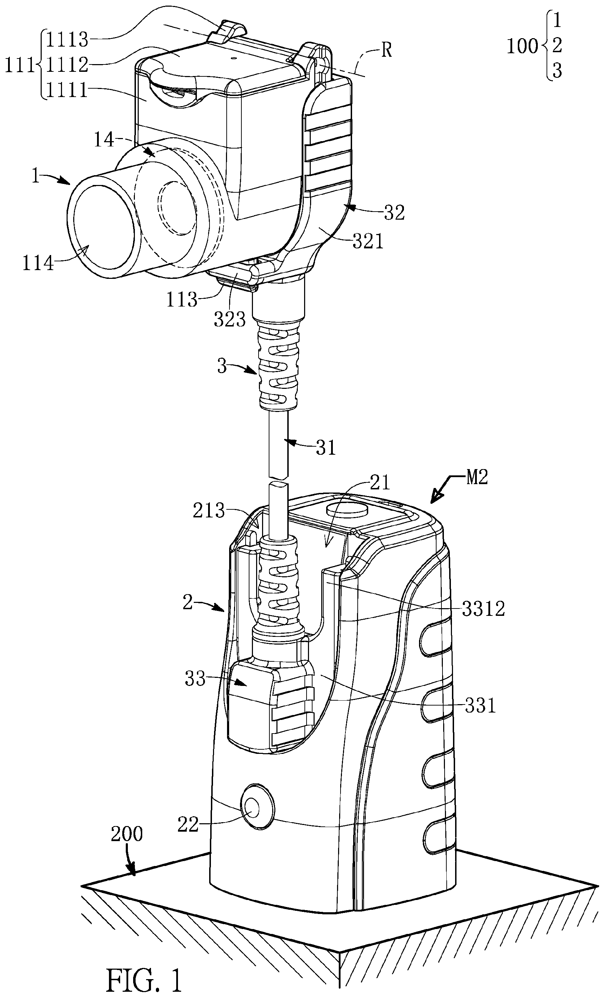

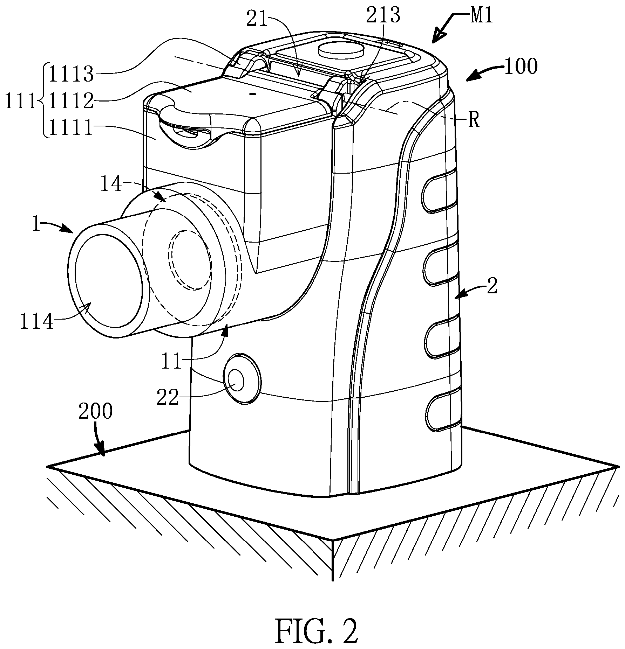

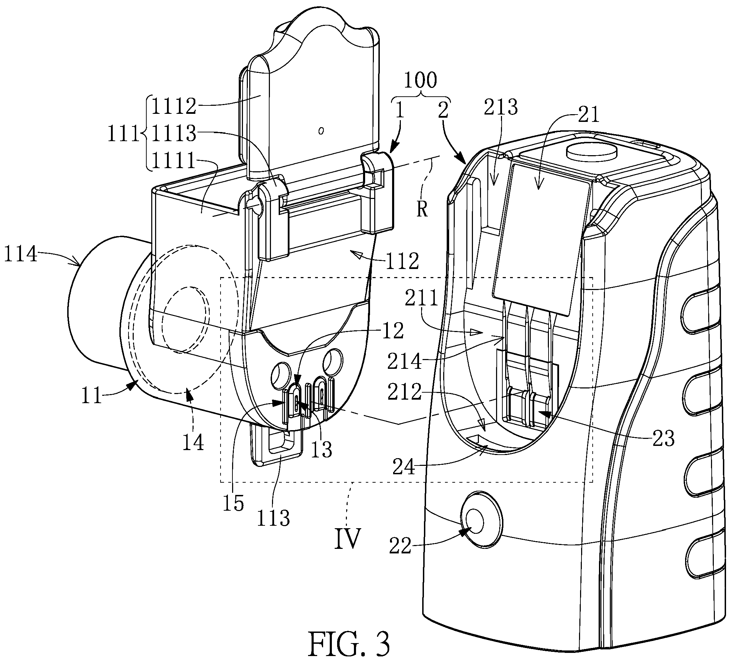

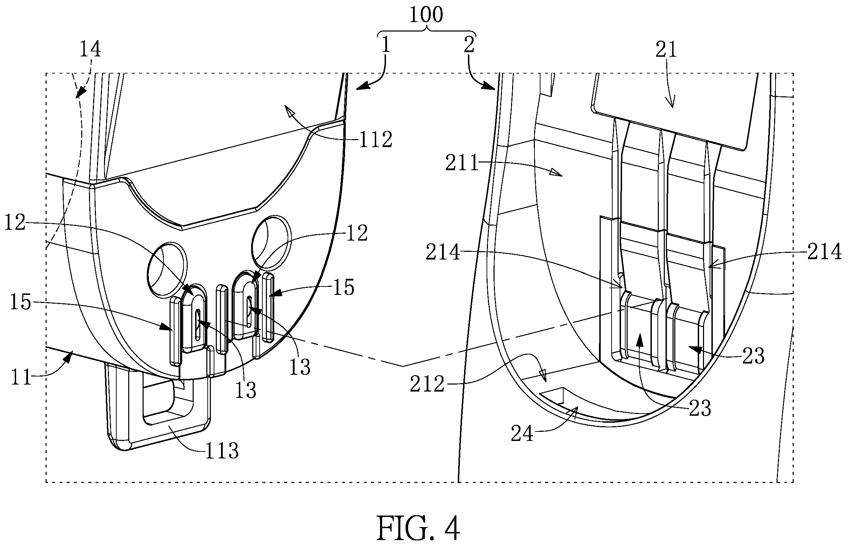

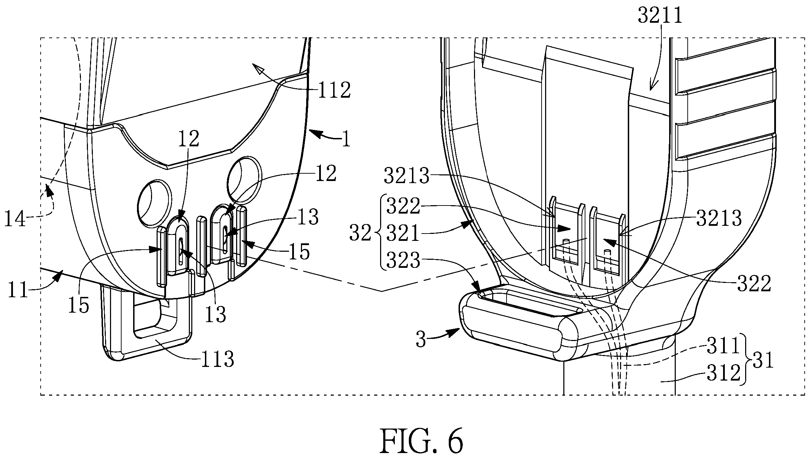

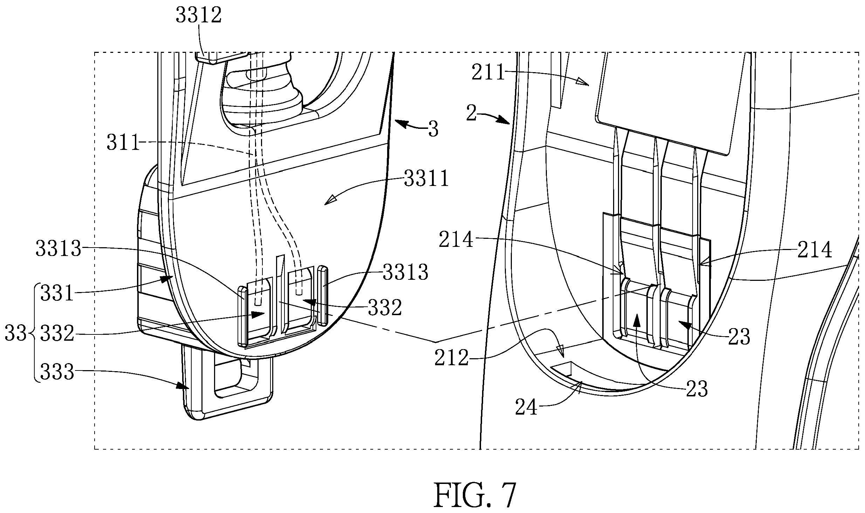

Please refer to FIG. 1 to FIG. 8, where a first embodiment of the present invention is provided. As shown in FIG. 1 and FIG. 2, the present embodiment provides a multi-mode atomization device 100, which includes an atomizer 1, a fixed-point drive station 2 electrically coupled to the atomizer 1, and a mode-switching member 3 that is selectively connected in-between the atomizer 1 and the fixed-point drive station 2. It should be noted that the mode-switching member 3 in the present embodiment is described in cooperation with the atomizer 1 and the fixed-point drive station 2, but the present invention is not limited thereto. For example, in other embodiments of the present invention not shown in the drawings, the mode-switching member 3 can also be independently used (e.g., sold) or can be used in cooperation with other components.

In the present embodiment, the multi-mode atomization device 100 is selectively in an upright operation mode M1 (e.g., FIG. 2) by inserting the atomizer 1 into the fixed-point drive station 2, or in an extension operation mode M2 (e.g., FIG. 1) by respectively assembling the mode-switching member 3 to the atomizer 1 and the fixed-point drive station 2. For ease of understanding, the following description describes the structure of the atomizer 1 and the fixed-point drive station 2 that are in the upright operation mode M1, and then describes the multi-mode atomization device 100 in the extension operation mode M2 provided by assembling the mode-switching member 3 to the atomizer 1 and the fixed-point drive station 2.

As shown in FIG. 2 to FIG. 4, the atomizer 1 includes a carrier 11, a plurality of buffer pads 12 disposed on the carrier 11, a plurality of first contacts 13 that are fixed to the carrier 11 and that are respectively inserted into the buffer pads 12, and an atomization module 14 that is assembled to the carrier 11, but the present invention is not limited thereto. For example, in other embodiments of the present invention not shown in the drawings, the buffer pads 12 can be omitted or can be replaced by other components according to design requirements.

In the present embodiment, the carrier 11 has a liquid storage chamber 111, a matching surface 112 located at one side of the liquid storage chamber 111, a positioning sheet 113 arranged adjacent to the matching surface 112, and an atomization outlet 114 that is located at another side of the liquid storage chamber 111. The liquid storage chamber 111 is configured to accommodate a liquid to be atomized (e.g., a medicine solution), and the liquid storage chamber 111 has a container 1111 and a sealing cap 1112 that is pivotally connected to the container 1111 (along the rotation axis R). The sealing cap 1112 rotatably covers (an opening of) the container 1111 to prevent the liquid to be atomized from flowing out of the liquid storage chamber 111 through the opening of the container 1111.

Specifically, the container 1111 is trough-shaped and a bottom of the container 1111 is in spatial communication with the atomization outlet 114 having a tubular shape, and the container 1111 has two positioning blocks 1113 connected to the matching surface 112. The two positioning blocks 1113 are substantially located at two corners of a top portion of the matching surface 112, and the sealing cap 1112 is pivotally connected to the two positioning blocks 1113 along the rotation axis R. It should be noted that, whether the multi-mode atomization device 100 in the present embodiment is in the upright operation mode M1 (as shown in FIG. 2) or the extension operation mode M2 (as shown in FIG. 1), the sealing cap 1112 is rotatable by at least 90 degrees relative to the container 1111, so as to facilitate the increase or decrease of the liquid to be atomized in the container 1111.

The positioning sheet 113 is connected to the bottom side of the carrier 11 and is substantially in a shape of a square ring, and the positioning sheet 113 is arranged adjacent to the first contacts 13 and the buffer pads 12. The buffer pads 12 are assembled on the matching surface 112, and the first contacts 13 are exposed from the matching surface 112. In the present embodiment, each of the first contacts 13 is a metal wire that is inserted into one of the buffer pads 12, and a portion of each of the first contacts 13 preferably protrudes outward from a corresponding one of the buffer pads 12, so as to enable each of the first contacts 13 to be in elastic contact with another corresponding component. The portion of each of the first contacts 13 can be bent into various shapes (e.g., an arc shape, a polygon shape, or an irregular shape) according to design requirements, and the present invention is not limited thereto.

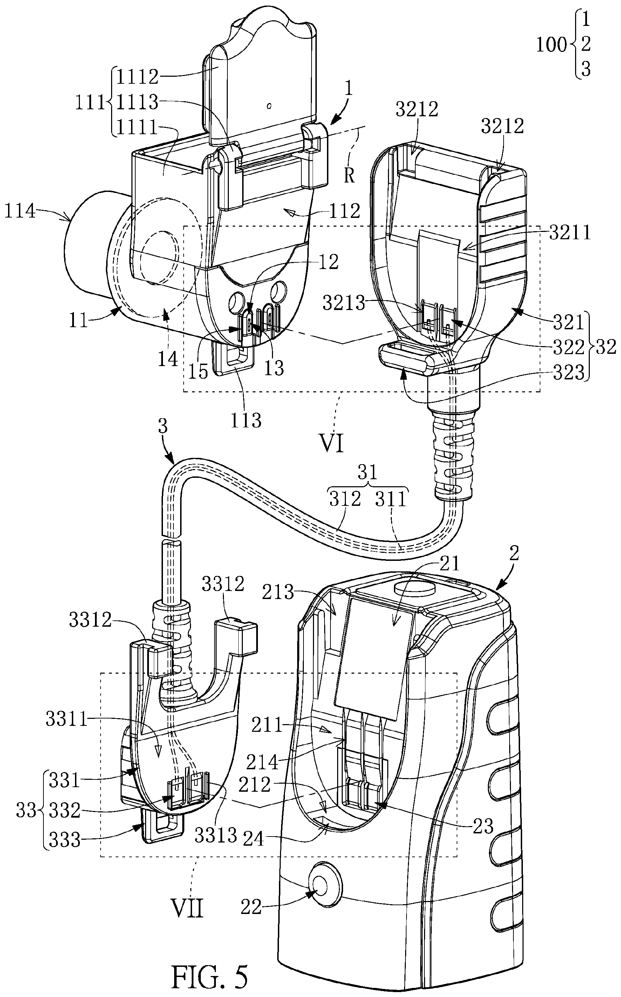

In addition, in order to enable the atomizer 1 to be quickly and accurately assembled to the fixed-point drive station 2 or the mode-switching member 3 (as shown in Figure 5), the atomizer 1 in the present embodiment further has a plurality of ribs 15 formed on the matching surface 112, and any one of the buffer pads 12 and the corresponding first contact 13 are located between two of the adjacent ribs 15 adjacent to each other, thereby effectively ensuring that the first contacts 13 are only in contact with the corresponding components.

The opposite sides of the atomization module 14 are respectively in spatial communication with the liquid storage chamber 111 and the atomization outlet 114, and the specific structure of the atomization module 14 can be adjusted or changed according to the design requirements and is not limited by the present embodiment. In order to facilitate the understanding of the operation of the present embodiment, the atomization module 14 in the following description is described as a piezoelectric (PZT) atomization module, which includes a carrying ring, an atomization film assembled to the carrying ring, and a piezoelectric ring that is fixed to the carrying ring. The piezoelectric ring can vibrate the carrying ring and the atomization film, so that when the liquid to be atomized in the liquid storage chamber 111 passes through the atomization film through the vibration of the atomization film, the liquid to be atomized becomes a plurality of atomized particles entering the atomization outlet 114.

The fixed-point drive station 2 includes an engaging slot 21, a locking member 22 located outside of the engaging slot 21, and a plurality of second contacts 23 that are exposed from the engaging slot 21. The engaging slot 21 includes an inner side wall 211 and an inner bottom wall 212 that is substantially U-shaped and that is perpendicularly connected to a periphery of the inner side wall 211. The engaging slot 21 (e.g., the inner side wall 211) has two positioning notches 213 respectively recessed in two corners thereof, and the fixed-point drive station 2 has a locking slot 24 recessed in the inner bottom wall 212.

Specifically, the locking member 22 is assembled in the locking slot 24 to lock a component inserted in the locking slot 24, and the specific structure of the locking member 22 can be adjusted or changed according to design requirements, and the present invention is not limited thereto. Furthermore, each of the second contacts 23 in the present embodiment is a copper pad, and the second contacts 23 are exposed from a bottom of the inner side wall 211, and the second contacts 23 are arranged adjacent to the locking slot 24 and away from the two positioning notches 213.

In addition, in order to enable the fixed-point drive station 2 to be quickly and accurately assembled to the atomizer 1 or the mode-switching member 3 (e.g., FIG. 5), the fixed-point drive station 2 in the present embodiment has a plurality of guiding grooves 214 recessed in the inner side wall 211, and any one of the second contacts 23 is located between two of the guiding grooves 214 adjacent to each other.

It should be noted that an interior of the fixed-point drive station 2 is configured to allow at least one battery to be assembled therein, so that the second contacts 23 can obtain power required for the operation of the multi-mode atomization device 100 from the at least one battery. In the present embodiment, the fixed-point drive station 2 can allow the at least one battery to be assembled from a bottom side thereof, and a start button is provided on the top side of the fixed-point drive station 2 for starting or stopping the operation of the fixed-point drive station 2.

In summary, as shown in FIG. 2 to FIG. 4, when the multi-mode atomization device 100 is in the upright operation mode M1, the carrier 11 is inserted into the engaging slot 21, and the first contacts 13 are respectively in contact with the second contacts 23, so that the atomizer 1 and the fixed-point drive station 2 are capable of standing on a working surface 200 (e.g., a desktop) through a center of gravity thereof. In other words, any driver that cannot operate in a standing manner is different from the fixed-point drive station 2 provided by the present embodiment.

Specifically, when the multi-mode atomization device 100 is in the upright operation mode M1, the positioning sheet 113 of the carrier **11** is inserted into the locking slot 24 of the fixed-point drive station 2, so that the positioning sheet 113 and the locking member 22 are engaged with each other for jointly being in a locked state, and the locking member 22 is pressable to separate from the positioning sheet 113, thereby enabling the locking member 22 and the positioning sheet 113 to be changed from the locked state to an unlocked state.

Furthermore, when the multi-mode atomization device 100 is in the upright operation mode M1, the two positioning blocks 1113 are respectively engaged in the two positioning notches 213, the rotation axis R is located within the engaging slot 21 of the fixed-point drive station 2, and the ribs 15 are respectively inserted into the guiding grooves 214, so that the portion of any one of the first contacts 13 protruding from the corresponding buffer pad 12 can elastically abut against one of the second contacts 23.

It should be noted that the two positioning blocks 1113, the multiple ribs 15, and the positioning sheet 113 of the atomizer 1 in the present embodiment are respectively located on different planes and are preferably respectively fixed to the two positioning notches 213, the guiding grooves 214, and the locking slot 24 of the fixed-point drive station 2, so as to effectively enhance a positioning effect between the atomizer 1 and the fixed-point drive station 2, but the present invention is not limited thereto.

As shown in FIG. 1 and FIG. 5 to FIG. 7, the mode-switching member 3 includes a transmission cable 31, a handheld holder 32 connected to one end of the transmission cable 31, and a connector 33 that is connected to another end of the transmission cable 31. The transmission cable 31 includes a plurality of conductive wires 311 and an insulating layer 312 that covers the conductive wires 311, and the handheld holder 32 and the connector 33 can be electrically coupled to each other through the conductive wires 311.

Accordingly, the multi-mode atomization device 100 can selectively use the handheld holder 32 and the connector 33 of the mode-switching member 3 to be respectively connected to the atomizer 1 and the fixed-point drive station 2 for being in the extension operation mode M2, and the hand-held stand 32 needs to have a carrying function and a hand-operating function for the atomizer 1, such that any transmission cable that does not have the above function is different from the mode-switching member 3 provided by the present embodiment.

Specifically, the handheld holder 32 includes a seat 321, a plurality of first metal pads 322 fixed to the seat 321, and a locking ring 323 that is connected to the seat 321. The seat 321 is integrally connected to one end of the insulating layer 312 and has an imitation engaging slot 3211, and the shape of the imitation engaging slot 3211 is similar to or substantially the same as the shape of the engaging slot 21, so that the imitation engaging slot 3211 has a same function as the engaging slot 21.

In the present embodiment, the imitation engaging slot 3211 has two imitation positioning notches 3212 respectively recessed in at two corners thereof, and the imitation engaging slot 3211 further has a plurality of imitation guiding grooves 3213 that are recessed therein and that are arranged away from the two imitation positioning notches 3212. The shape of the imitation positioning notch 3212 is similar to or substantially same as the shape of the positioning notch 213, and the shape of the imitation guiding groove 3213 is similar to or substantially same as the shape of the guiding groove 214, but the present invention is not limited thereto.

Furthermore, the shapes and arrangement of the first metal pads 322 are identical to those of the second contacts 23, and the first metal pads 322 are respectively electrically coupled to the conductive wires 311. The first metal pads 322 are exposed from the imitation engaging slot 3211, and any one of the first metal pads 322 is located between two of the imitation guiding grooves 3213 adjacent to each other. In other words, the first metal pads 322 are arranged away from the two imitation positioning notches 3212, but are arranged adjacent to the locking ring 323.

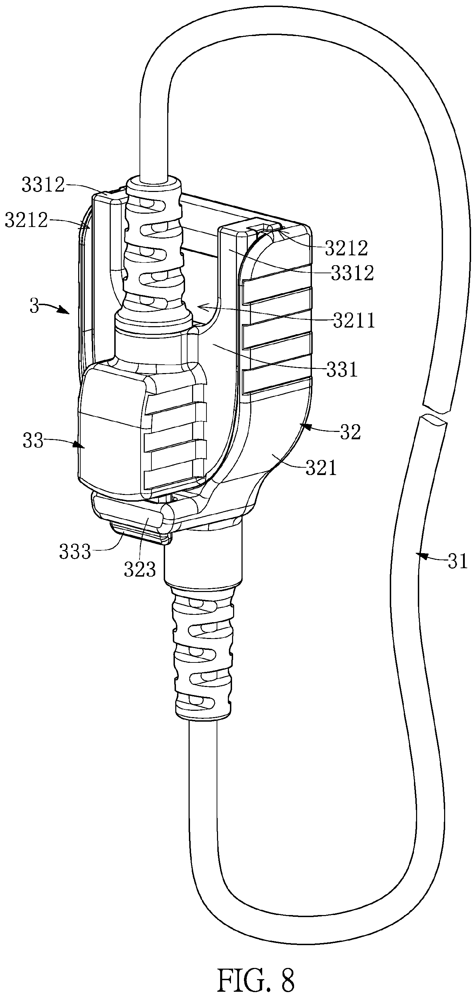

The connector 33 includes an engaging body 331, a plurality of second metal pads 332 fixed to the engaging body 331, and an imitation positioning sheet 333 that is connected to the engaging body 331. The engaging body 331 of the connector 33 corresponds in geometric shape to and is detachably inserted into the imitation engaging slot 3211 of the handheld holder 32 (as shown in FIG. 8). In other words, the configuration of the engaging body 331 is substantially complementary to the configuration of the imitation engaging slot 3211. Accordingly, the engaging body 331 of the mode-switching member 3 can be inserted into the imitation engaging slot 3211 for enabling the mode-switching member 3 to form an enclosed ring structure, so that the interfaces that are easy to receive and cooperate with each other can effectively avoid being damaged by impact, but the present invention is not limited thereto.

Specifically, the engaging body 331 is integrally connected to another end of the insulating layer 312 and has an imitation matching surface 3311, and the engaging body 331 includes two imitation positioning blocks 3312 connected to the imitation matching surface 3311 and two imitation ribs 3313 that are formed on the matching surface 112. The imitation matching surface 3311 is substantially U-shaped, and the two imitation positioning blocks 3312 are respectively arranged on the two ends of the imitation matching surface 3311, and the two imitation ribs 3313 are arranged away from the two imitation positioning blocks 3312.

In other words, the shape of the imitation matching surface 3311 is similar to or substantially the same as the shape of the matching surface 112, the shape of the imitation positioning block 3312 is similar to or substantially the same as the shape of the positioning block 1113, and the imitation rib 3313 is similar to or substantially the same as the shape of the matching surface 112, but the present invention is not limited thereto.

Furthermore, the second metal pads 332 are exposed from the imitation matching surface 3311, and the second metal pads 332 are electrically coupled to the conductive wires 311, so that each of the first metal pads 322 is electrically coupled to a corresponding one of the second metal pad 332 through one of the conductive wires 311. The second metal pads 332 are located between the two imitation ribs 3313 and are arranged away from the two imitation positioning blocks 3312.

In addition, the imitation positioning sheet 333 is connected to an end edge of the engaging body 331 and is substantially in a shape of a square ring, and the imitation positioning sheet 333 is arranged adjacent to the second metal pads 332. The shape of the imitation positioning sheet 333 is similar to or substantially the same as the shape of the positioning sheet 113, so as to have substantially a same function.

In summary, as shown in FIG. 1 and FIG. 5 to FIG. 7, when the multi-mode atomization device 100 is in the extension operation mode M2, the carrier 11 of the atomizer 1 is inserted into the imitation engaging slot 3211 of the handheld holder 32 (e.g., the imitation engaging slot 3211 substantially covering at least 80% of an area of the matching surface 112 to facilitate hand-held operation of the handheld holder 32), the first contacts 13 respectively abut against the first metal pads 322 (e.g., the portion of any one of the first contacts 13 protruding from the corresponding buffer pad 12 elastically abutting against one of the first metal pads 322), the engaging body 331 of the connector 33 is inserted into the engaging slot 21 of the fixed-point drive station 2, and the second metal pads 332 respectively abut against the second contacts 23, so that the atomizer 1 is electrically coupled to the fixed-point drive station 2 through the mode-switching member 3 and is movable relative to the fixed-point drive station 2.

Accordingly, the multi-mode atomization device 100 in the present embodiment can selectively use the mode-switching member 3 to facilitate switching between the upright operation mode M1 (as shown in FIG. 2) and the extension operation mode M2 (as shown in FIG. 1) according to different requirements of the user. The mode-switching member 3 of the multi-mode atomization device 100 can also imitate part of the appearance of the fixed-point drive station 2 through the handheld holder 32, thereby having the carrying function and a hand-holding function for the atomizer 1, so as to enable the user to operate the atomizer 1 more stably.

Specifically, when the multi-mode atomization device 100 is in the extension operation mode M2, the positioning sheet 113 of the atomizer 1 is inserted into the locking ring 323 of the handheld holder 32, and the two positioning blocks 1113 are respectively engaged in the two imitation positioning notches 3212, but the rotation axis R is exposed from the imitation engaging slot 3211 of the handheld holder 32, and the ribs 15 are respectively inserted into the imitation guiding grooves 3213.

Furthermore, when the multi-mode atomization device 100 is in the extension operation mode M2, the imitation positioning sheet 333 of the connector 33 is inserted into the locking slot 24 of the fixed-point drive station 2, the two imitation positioning blocks 3312 are respectively engaged with the two positioning notches 213, and the two imitation ribs 3313 are respectively inserted into the two guiding slots 214.

It should be noted that the two positioning blocks 1113, the ribs 15, and the positioning sheet 113 of the atomizer 1 in the present embodiment are respectively located on different planes and are respectively fixed to the two imitation positioning notches 3212, the imitation guiding grooves 3213, and the locking ring 323 of the handheld holder 32, so as to effectively enhance the positioning effect between the atomizer 1 and the handheld holder 32, but the present invention is not limited thereto.

### [Second embodiment]

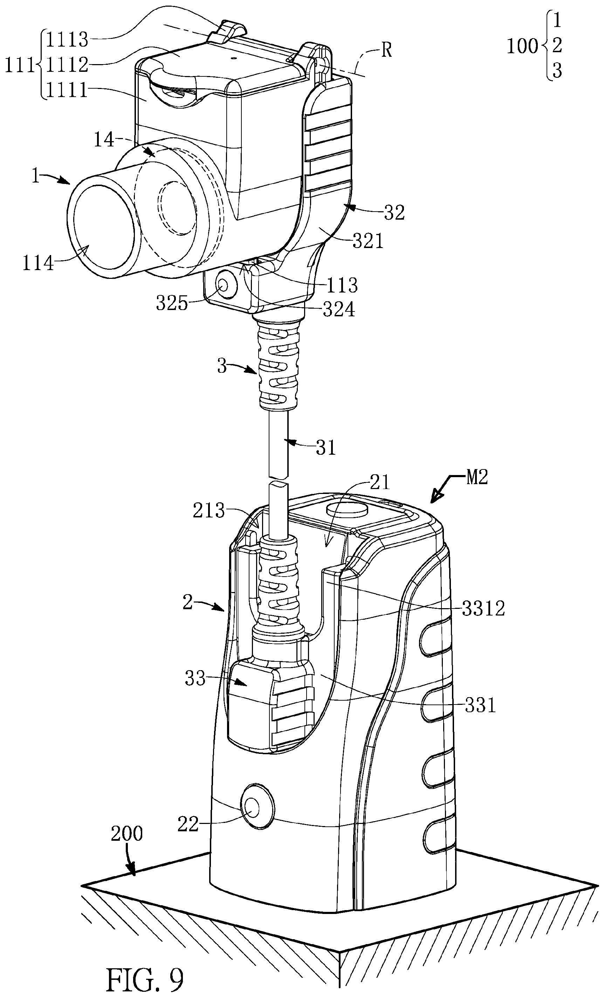

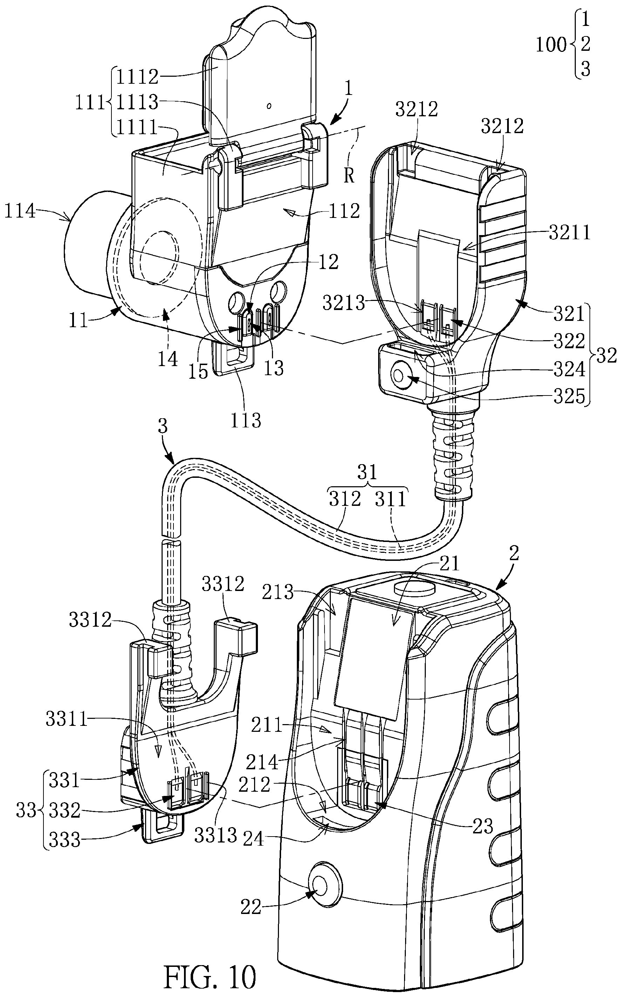

Please refer to FIG. 9 and FIG. 10, which show a second embodiment of the present invention. Since the present embodiment is similar to the first embodiment, descriptions of same components in the first and second embodiments of the present invention will be omitted herein for the sake of brevity, and the following description discloses different features between the first and second embodiments.

In the present embodiment, the handheld holder 32 includes an insertion slot 324 that is connected to the seat 321 and that is arranged adjacent to the first metal pads 322 and a button 325 that is assembled in the insertion slot 324. In other words, the handheld holder 32 in the present embodiment replaces the locking ring 323 of the first embodiment by the insertion slot 324 and the button 325.

Furthermore, when the multi-mode atomization device 100 is in the extension operation mode M2, the imitation positioning sheet 333 of the connector 33 is inserted into the locking slot 24 of the fixed-point drive station 2, the positioning sheet 113 of the carrier 11 is inserted into the insertion slot 324 and is interlocked with the button 325, and the button 325 is pressable to separate from the positioning sheet 113.

Accordingly, the mode-switching member 3 can effectively improve the connection stability between the atomizer 1 and the handheld holder 32 by providing the insertion slot 324 and the button 325.

### [Beneficial Effects of the Embodiments of the present invention]

In summary, the multi-mode atomization device disclosed in the embodiments of the present invention can selectively use the mode-switching member to facilitate switching between the upright operation mode and the extension operation mode according to different requirements of the user. Furthermore, the mode-switching member of the multi-mode atomization device can also imitate part of the appearance of the fixed-point drive station through the handheld holder, thereby providing the carrying function and the hand-holding function for the atomizer for enabling the user to operate the atomizer more stably.

The contents disclosed above are only preferred and feasible embodiments of the present invention, and do not limit the scope of the present invention. Therefore, all equivalent technical changes made using the contents of the description and drawings of the present invention are included in the scope of the present invention.

## Claims

1. A multi-mode atomization device, **characterized in that** the multi-mode atomization device comprises:
an atomizer comprising:
a carrier having a liquid storage chamber, a matching surface located on one side of the liquid storage chamber, and an atomization outlet that is located on another side of the liquid storage chamber;
a plurality of first contacts fixed to the carrier and exposed from the matching surface;
an atomization module mounted on the carrier, wherein two opposite sides of the atomization module are in spatial communication with the liquid storage chamber and the atomization outlet, respectively; and
a fixed-point drive station including an engaging slot and a plurality of second contacts exposed from the engaging slot; and
a mode-switching member including:
a transmission cable including a plurality of conductive wires and an insulating layer that covers the plurality of conductive wires;
a handheld holder including:
a seat integrally connected to one end of the insulating layer and having an imitation engaging slot; and
a plurality of first metal pads fixed to the seat and exposed from the imitation engaging slot, wherein the plurality of first metal pads are electrically coupled to the plurality of conductive wires, respectively; and
a connector including:
an engaging body integrally connected to another end of the insulating layer and having an imitation matching surface; and
a plurality of second metal pads fixed to the engaging body and exposed from the imitation matching surface, wherein the plurality of second metal pads are electrically coupled to the plurality of conductive wires, respectively, so that each of the first metal pads is electrically coupled to one of the second metal pads through one of the conductive wires;
wherein the multi-mode atomization device is selectively in an upright operation mode by inserting the atomizer into the fixed-point drive station, or in an extension operation mode by respectively assembling the handheld holder and the connector of the mode-switching member to the atomizer and the fixed-point drive station;
wherein, when the multi-mode atomization device is in the upright operation mode, the carrier is inserted into the engaging slot, and the first contacts respectively abut against the second contacts, so that the atomizer and the fixed-point drive station are capable of standing on a working surface through a center of gravity thereof;
wherein, when the multi-mode atomization device is in the extension operation mode, the carrier is inserted into the imitation engaging slot, and the plurality of first contacts respectively abut against the plurality of first metal pads, and the engaging body is inserted into the engaging slot, and the plurality of second metal pads respectively abut against the plurality of second contacts, so that the atomizer is configured to be movable relative to the fixed-point drive station through the mode-switching member.

2. The multi-mode atomization device according to claim **1, characterized in that** the carrier has a positioning sheet adjacent to the matching surface, and the fixed-point drive station has a locking slot that is recessed in an inner bottom wall of the engaging slot and that is arranged adjacent to the plurality of second contacts, wherein, when the multi-mode atomization device is in the upright operation mode, the positioning sheet of the carrier is inserted into the locking slot of the fixed-point drive station.

3. The multi-mode atomization device according to claim 2, **characterized in that** the fixed-point drive station includes a locking member assembled in the locking slot, wherein, when the multi-mode atomization device is in the upright operation mode, the positioning sheet is inserted into the locking slot and is engaged with the locking member for jointly being in a locked state, and the locking member is pressable to separate from the positioning sheet, thereby enabling the locking member and the positioning sheet to be changed from the locked state to an unlocked state.

4. The multi-mode atomization device according to claim 2, **characterized in that** the handheld holder includes a that is connected to the seat and that is arranged adjacent to the plurality of first metal pads, and the connector includes an imitation positioning sheet connected to the engaging body, wherein, when the multi-mode atomization device is in the extension operation mode, the positioning sheet of the carrier is inserted into the locking ring of the handheld holder, and the imitation positioning sheet of the connector is inserted into the locking slot of the fixed-point drive station.

5. The multi-mode atomization device according to claim 2, **characterized in that** the handheld holder includes an insertion slot that is connected to the seat and that is arranged adjacent to the plurality of first metal pads and a button that is assembled in the insertion slot, and the connector includes an imitation positioning sheet connected to the engaging body, wherein, when the multi-mode atomization device is in the extension operation mode, the imitation positioning sheet of the connector is inserted into the locking slot of the fixed-point drive station, the positioning sheet of the carrier is inserted into the insertion slot and is interlocked with the button, and the button is pressable to separate from the positioning sheet.

6. The multi-mode atomization device according to claim **1, characterized in that** the atomizer includes a plurality of buffer pads assembled on the matching surface, and each of the plurality of first contacts is a metal wire inserted into one of the plurality of buffer pads, wherein a part of each of the plurality of first contacts protrudes outward from a corresponding one of the plurality of buffer pads so as to be able to elastically abut against one of the plurality of second contacts or one of the plurality of first metal pads.

7. The multi-mode atomization device according to claim 6, **characterized in that** the atomizer has a plurality of ribs formed on the matching surface, and any one of the plurality of buffer pads and the corresponding first contact is located between two of the plurality of ribs adjacent to each other, the fixed-point drive station has a plurality of guiding grooves recessed in an inner side wall of the engaging slot, and any one of the plurality of second contacts is located between two of the plurality of guiding grooves adjacent to each other, wherein, when the multi-mode atomization device is in the upright operation mode, the plurality of ribs are respectively inserted into the plurality of guiding grooves.

8. The multi-mode atomization device according to claim 7, **characterized in that** the seat of the handheld holder has a plurality of imitation guiding grooves recessed in the imitation engaging slot, and any one of the plurality of first metal pads is located between two of the plurality of imitation guiding grooves adjacent to each other, wherein the engaging body of the connector includes two imitation ribs formed on the matching surface, and the plurality of second metal pads are located between the two imitation ribs, and wherein, when the multi-mode atomization device is in the extension operation mode, the plurality of ribs are respectively inserted into the plurality of imitation guiding grooves, and the two imitation ribs are respectively inserted into two of the plurality of guiding grooves.

9. The multi-mode atomization device according to claim **1, characterized in that** the carrier has two positioning blocks connected to the matching surface and arranged on the liquid storage chamber, and the fixed-point drive station has two positioning notches that are respectively recessed in two corners of the engaging slot and that are arranged away from the plurality of second contacts, wherein, when the multi-mode atomization device is in the upright operation mode, the two positioning blocks are respectively engaged with the two positioning notches.

10. The multi-mode atomization device according to claim 9, **characterized in that** the seat of the handheld holder has two imitation positioning notches that are respectively recessed in two corners of the imitation engaging slot and that are arranged away from the plurality of first metal pads, wherein the engaging body has two imitation positioning blocks connected to the imitation matching surface, and wherein, when the multi-mode atomization device is in the extension operation mode, the two positioning blocks are respectively engaged in the two imitation positioning notches, and the two imitation positioning blocks are respectively engaged in the two positioning notches.

11. The multi-mode atomization device according to claim 1, **characterized in that** the liquid storage chamber of the atomizer has a container and a sealing cap that is pivotally connected to the container and that rotatably closes the container, wherein, when the multi-mode atomization device is in the upright operation mode or the extension operation mode, the sealing cap is rotatable by at least 90 degrees relative to the container.

12. The multi-mode atomization device according to claim 11, **characterized in that** the sealing cap is pivotally connected to the container along a rotation axis, wherein, when the multi-mode atomization device is in the upright operation mode, the rotation axis is located within the engaging slot of the fixed-point drive station.

13. The multi-mode atomization device according to claim 12, **characterized in that**, when the multi-mode atomization device is in the extension operation mode, the rotation axis is exposed from the imitation engaging slot of the handheld holder.

14. The multi-mode atomization device according to claim **1, characterized in that** the engaging body of the connector corresponds in geometric shape to and is detachably inserted into the imitation engaging slot of the handheld holder.

15. A mode-switching member of a multi-mode atomization device, **characterized in that** the mode-switching member of the multi-mode atomization device comprises:
a transmission cable including a plurality of conductive wires and an insulating layer that covers the plurality of conductive wires;
a handheld holder including:
a seat integrally connected to one end of the insulating layer and having an imitation engaging slot; and
a plurality of first metal pads fixed to the seat and exposed from the imitation engaging slot, wherein the plurality of first metal pads are electrically coupled to the plurality of conductive wires, respectively; and
a connector including:
an engaging body integrally connected to another end of the insulating layer and having an imitation matching surface; and
a plurality of second metal pads fixed to the engaging body and exposed from the imitation matching surface, wherein the plurality of second metal pads are electrically coupled to the plurality of conductive wires, respectively, so that each of the plurality of first metal pads is electrically coupled to one of the plurality of second metal pads through one of the plurality of conductive wires;
wherein the handheld holder and the connector of the mode-switching member are configured to allow an atomizer and a fixed-point drive station to be respectively connected thereto, so that the atomizer and the fixed-point drive station assembled to each other and in an upright operation mode are adjustable to be separated from each other for being in an extension operation mode.

16. The mode-switching member of the multi-mode atomization device according to claim 15, **characterized in that** the engaging body of the connector corresponds in geometric shape to and is detachably inserted into the imitation engaging slot of the handheld holder.

17. The mode-switching member of the multi-mode atomization device according to claim 15, **characterized in that** the handheld holder includes an insertion slot that is connected to the seat and that is arranged adjacent to the plurality of first metal pads and a button that is assembled in the insertion slot, and the connector includes an imitation positioning sheet connected to the engaging body.
